# EUROPÄISCHE PATENTSCHRIFT

(11) **EP 0 606 616 B1**
(45) Veröffentlichungstag und Bekanntmachung des Hinweises auf die Patenterteilung: **10.09.1997**
(21) Anmeldenummer: 93120470.5
(22) Anmeldetag: 18.12.1993
(51) Int. Cl.: B01J 20/32, B01D 15/08

(54) **Hydrophobe Adsorbentien und ihre Verwendung zur Adsorption von Lipoproteinen**
Hydrophobic adsorbents and their use for lipoproteins adsorption
Adsorbants hydrophobes et leur utilisation pour l'adsorption de lipoproteines

(30) Priorität: 09.01.1993 DE 4300412
(43) Veröffentlichungstag der Anmeldung: 20.07.1994
(73) Patentinhaber: BEHRINGWERKE Aktiengesellschaft, 35001 Marburg (DE)
(72) Erfinder: Merle, Peter, D-35041 Marburg (DE)

(56) Entgegenhaltungen:
- EP-A- 0 263 184
- EP-A- 0 424 698
- WO-A-91/19565
- GB-A- 1 066 459
- US-A- 4 029 583
- US-A- 4 696 958
- US-A- 5 030 352
- PATENT ABSTRACTS OF JAPAN vol. 9, no. 210 (P-383) & JP-A-60 070 353 (TOYO SODA) 22. April 1985
- JOURNAL OF CHROMATOGRAOHY Bd. 101 , 1974 Seiten 281 - 288 STELLAN HJERTEN 'HYDROPHOBIC INTERACTION CHROMATOGRAPHY'

## Beschreibung

Die Erfindung betrifft Adsorbentien bestehend aus einem zur Chromatographie geeigneten Polysaccharid-Träger, vorzugsweise auf der Basis von Agarose, der in einer chemischen Reaktion mit den Glycidylethern von nichtonischen Polyoxyethylen-Detergentien des Typs HO-(CH₂CH₂O)ₙ-Y-R umgesetzt wird, ein Verfahren zu ihrer Herstellung und ihre Verwendung zur Entfernung von Lipoproteinen aus menschlichem oder tierischen Körperflüssigkeiten.

Werden Körperflüssigkeiten wie beispielsweise Serum, Plasma, Liquor, Pleuralexudat oder Ascites menschlichen oder tierischen Ursprungs gelagert, so kann es zu Abscheidungen oder Eintrübungen kommen, welche die Qualität dieser Produkte erheblich verschlechtern. Mitverantwortlich für diese Instabilitäten werden die in Körperflüssigkeiten vorkommenden Lipoprotein-Makromoleküle gemacht, die aufgrund ihres Gehalts an wasserunlöslichen Phospholipiden per se eine Tendenz zur Aggregation aufweisen.

Entfernt man die Lipoproteine aus obengenannten Flüssigkeiten, so wird in der Regel eine gute Lagerstabilität erreicht.

Desweiteren sind eine Reihe von Tests in der medizinischen Diagnostik störanfällig, wenn hoch-lipoproteinhaltige Patientenproben zum Einsatz kommen.

Auch die Aufarbeitung von biologischen Materialien zu Arzneimitteln oder diagnostischen Testkomponenten kann in Gegenwart von Lipoproteinen erschwert oder verhindert werden.

In der Vergangenheit hat es nicht an Versuchen gefehlt, geeignete Verfahren zur Abtrennung von Lipoproteinen, vor allem aus Seren oder Plasmen, zu entwickeln. Diese bekannten Verfahren weisen jedoch Nachteile auf, die ihre industrielle Anwendung einschränken oder unmöglich machen:
a) Die Extraktion mit flüssigen Halogenkohlenwasserstoffen (z. B. Lipoclean^{R}) ist aus Umweltschutzgründen bedenklich.
b) Die Absorption mit Dextransulfat ist nicht quantitativ für alle Lipoproteine.
c) Octyl- oder Phenyl-Sepharose^{R} hat eine geringe Kapazität und ist nur mit einer aufwendigen Prozedur zu regenerieren.
d) Das in EP 137221 beschriebene Verfahren mit einem Polyhydroxymethylen-Derivat ist für den Prozess in Chromatographie-Säulen nur mit Einschränkung geeignet, das Material läßt sich nur schwer regenerieren.
e) Verfahren, die mit Siliciumdioxid-Adsorbentien arbeiten, sind für Plasma ungeeignet, da auch gerinnungsaktive Proteine, wie z. B. Fibrinogen, gebunden werden.
f) Die Methode der Flotation von Lipoproteinen in der Ultrazentrifuge erfordert eine sehr teure Ausrüstung und liefert nur einen geringen Mengendurchsatz.

Ziel der vorliegenden Erfindung war es, ein Adsorbens herzustellen, das es erlaubt, die Lipoproteine aus nativen biologischen Flüssigkeiten unter hohem Mengendurchsatz quantitativ zu entfernen. Dabei sollte das Adsorbens leicht regenerierbar sein und reproduzierbare hohe Kapazitäten aufweisen.

Die im Regenerierprozess von dem Adsorbens desorbierten Lipoproteine sollten ohne Schädigung gewonnen werden können, so daß sie als Rohstoffe für die Herstellung von diagnostischen Tests oder therapeutischen Präparaten dienen könnten.

Gegenstand der vorliegenden Erfindung sind Adsorbentien zur Entfernung von Lipoproteinen aus menschlichen oder tierischen Körperflüssigkeiten, bestehend aus einem zur Chromatographie geeigneten Polysaccharid-Träger, vorzugsweise auf der Basis von Agarose, der in einer chemischen Reaktion mit den Glycidylethern von nichionischen Polyoxyethylen-Detergentien des Typs HO-(CH₂CH₂O)ₙ-O-R umgesetzt wurde.

Solche Adsorbentien können besonders durch die Formel I 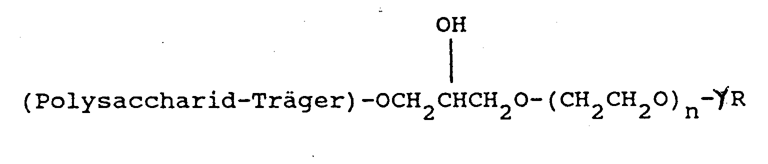 dargestellt werden, wobei
n eine ganze Zahl von 2 bis 30, vorzugsweise 6 bis 20, und
R ein Alkylrest mit 4-20, vorzugsweise mit 10-16 Kohlenstoffatomen oder ein Phenyl-Rest oder ein Phenyl-Alkyl-Rest ist, wobei der Alkylrest 1-16 Kohlenstoffatome hat und vorzugsweise tert.-octyl ist.

Das Träger-Polysaccharid ist vorzugsweise ein makroporöses Agarose-Gel mit einem Agarosegehalt von 1-6%. Vorteilhaft ist die Verwendung von Sepharose^{R} vom Typ CL-2B.

Geeignete Polyoxyethylen-Detergentien sind beispielsweise unter den Handelsnamen Brij^{R}, Genapol^{R}, Nonidet^{R}, Tergitol^{R} oder Triton^{R} bekannt. Diese Handelsprodukte sind nicht einheitlich und zeichnen sich durch eine Mischung von Ethern mit unterschiedlicher Polyoxyethylen-Kettenlänge aus. Von den Herstellern wird jeweils die Hauptkomponente oder ein Kettenlängen-Bereich angegeben. Die Struktur und Zusammensetzung solcher Polyoxyethylen-Detergentien ist in Methods in Enzymology, LVI, 734-749 beschrieben.

Die Glycidylether der Detergentien haben die allgemeine Formel II

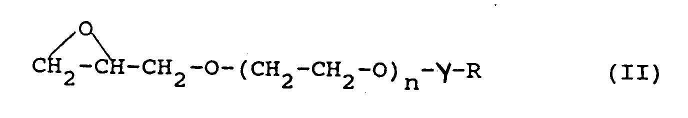

Gegenstand der Erfindung ist auch ein Verfahren zur Herstellung der erfindungsgemäßen Adsorbentien.

Die Umsetzung von Alkoholen oder Phenolen mit Epichlorhydrin zu den Alkyl- oder Phenyl-Glycidylethern ist in Journal of Chromatography, 101 (1974) 281-284 beschrieben. Dieses Verfahren kann für die Herstellung der Glycidylether der oben beschriebenen nichtionischen Detergentien verwandt werden. Dabei wird das nichtionische Detergenz in Gegenwart einer Lewissäure wie BF₃-etherat mit einem Epihalogenhydrin wie Epichlorhydrin erhitzt und anschließend in Gegenwart von NaOH bei Raumtemperatur zum Glycidylether-Derivat umgesetzt. Das Reaktionsschema ist nachfolgend allgemein beschrieben (HO-R¹ = Detergenz):

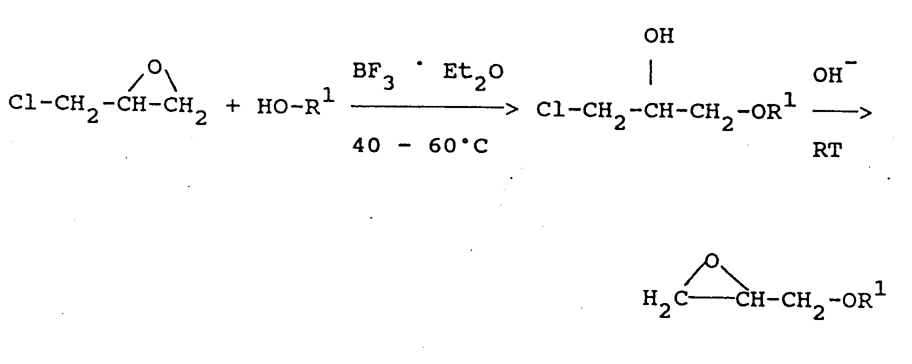

Für die Umsetzung der Glycidylether der nichtionischen Detergentien mit einem Polysaccharid kann das Verfahren für Alkyl- oder Phenylglycidylether, welches in Journal of Chromatography, 101 (1974) 281-288 beschrieben ist, genutzt werden. Dabei wird das entwässerte Polysaccharid, in wasserfreiem, inertem Lösemittel, beispielsweise Dioxan, suspendiert und in Gegenwart von BF₃-etherat mit dem Glycidylether eines nichtionischen Detergenz umgesetzt:

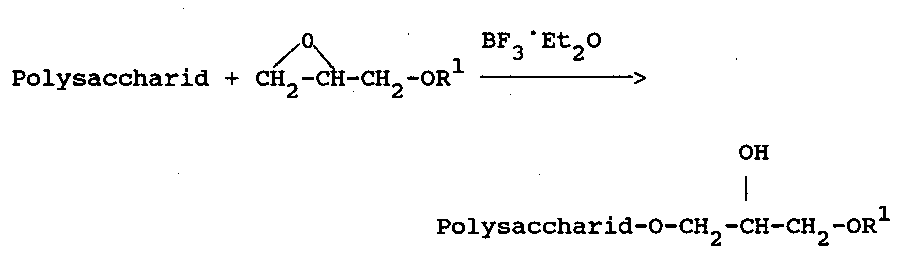

Das erfindungsgemäße Polysaccharidderivat (Adsorbens) wird in ein wässriges Medium überführt und ist fertig zum Gebrauch.

Zur Adsorption von Lipoproteinen aus biologischen Flüssigkeiten ist es vorteilhaft, die erfindungsgemäßen Adsorbentien in eine Chromatographie-Säule oder in eine Kartusche zu packen.

Jedoch ist es auch möglich, die Adsorption der Lipoproteine durch direkte Zugabe der Adsorbentien zu biologischen Flüssigkeiten durchzuführen. Dazu kann das Adsorbens auch in getrockneter Form zugegeben werden.

Nachdem die biologischen Flüssigkeiten mit einem erfindungsgemäßen Adsorbens in einem der oben erwähnten Verfahren in Kontakt gebracht wurden, werden sie als klare, lipoproteinfreie Flüssigkeiten gewonnen.

Die Lipoproteine können nach Adsorption an ein Adsorbens mit Lösungen von Substanzen, die hydrophobe Bindungen spalten, eluiert werden. Dies sind beispielsweise Lösungen von ionischen oder nichionischen Detergentien, von Harnstoff, KSCN oder Guanidin·HCL oder Lösungen von Alkoholen, wie Ethylenglycol.

Vorzugsweise sind solche Substanzen zu wählen, die keine Denaturierung der Lipoproteine provozieren. Eine schnelle und quantitative Elution wird erreicht, wenn eine Lösung des nichtionischen Detergenz verwendet wird, dessen Glycidylether zur Derivatisierung des Polysaccharids eingesetzt wurde.

### Beispiele

### 1. Herstellung des Glycidylethers von Genapol^{R} T250

10g Genapol^{R} T250 wurden in 40 ml wasserfreiem Dioxan gelöst und mit 1ml BF₃-Etherat versetzt. Der Ansatz wurde in einem Rundkolben mit Trockenrohr-Aufsatz und Magnetrührer auf 50°C erhitzt. Es wurden 2 ml Epichlorhydrin zugegeben und 2h bei 50°C gerührt.

Danach wurde der Ansatz auf Raumtemperatur abgekühlt und mit 4g NaOH-Plätzchen versetzt. Es wurde 1,5h bei Raumtemperatur gerührt.

Nach Zentrifugation des Ansatzes in einer Tischzentrifuge konnte die Lösung des Glycidylethers von Genapol^{R} T250 in Dioxan als Überstand gewonnen werden.

### 2. Entwässerung von Sepharose^{R} CL-2B

150 ml einer wässrigen Suspension von Sepharose^{R} CL-2B wurden in eine Glas-Chromatographie-Säule gefüllt und anschließend mit folgenden Lösungen im freien Fluß gespült:
a) 1 Säulenvolumen Wasser
b) danach ein Säulenvolumen 25% Ethanol in Wasser
c) danach ein Säulenvolumen 50% Ethanol in Wasser
d) danach ein Säulenvolumen 96% Ethanol in Wasser
e) abschließend 2 Säulenvolumina wasserfreies Dioxan

Die entwässerte Sepharose^{R} CL-2B wurde aus der Säule in ein Gefäß gefüllt und bis zu ihrer Verwendung unter Ausschluß von Feuchtigkeit gelagert.

### 3. Kopplung des Glycidylethers von Triton^{R} X-100 an Sepharose^{R} CL-2B

100g nach Beispiel 2 entwässerte Sepharose^{R} wurden in 100ml wasserfreiem Dioxan suspendiert und in einem geschlossenen Rührgefäß mit 2ml BF₃-etherat und 10 ml einer 20%igen Lösung des Glycidylethers von Triton^{R} X-100 in Dioxan versetzt und 2h langsam bei Raumtemperatur gerührt. Danach wurde das derivatisierte Gel in eine Glas-Chromatographie-Säule gefüllt und mit den folgenden Lösungen gespült:
a) ein Säulenvolumen Dioxan
   ein Säulenvolumen 96% Ethanol in Wasser
   ein Säulenvolumen 50% Ethanol in Wasser
   ein Säulenvolumen 25% Ethanol in Wasser

Das derivatisierte Gel wurde bis zu seiner Verwendung in 25% Ethanol bei 4°C aufbewahrt.

### 4. Adsorption der Lipoproteine aus Human-Citratplasma

Eine Chromatographie-Säule (4 X 30 cm) wurde mit 120 ml eines Triton^{R} X-100 Derivates von Sepharose^{R} CL-2B gefüllt und mit einem Puffer, bestehend aus 0,9% NaCl, 10mM Natriumcitrat, pH 7,0, nachfolgend Puffer A genannt, äquilibriert.

Die Säule wurde unter einem Fluß von 6ml/min mit 360 ml eines frischen Human-Citratplasmas beschickt. Es wurde mit Puffer A nachgespült.

Fraktionen mit maximaler Absorption wurden gesammelt. Es konnten 320 ml eines klaren Humanplasmas gewonnen werden in dem weder Cholesterin (Monotest^{R} Cholesterin, Boehringer: < 2,5mg/dl) noch Apolipoprotein A-I und Apolipoprotein B (nephelometrische Tests, Behringwerke AG: ApoAl <5mg/dl; ApoB <7,3mg/dl) nachgewiesen werden konnten.

### 5. Delipidierung von Kaninchenserum

400ml Kaninchenserum wurden über eine Säule nach Beispiel 4 gegeben, wobei ein Fluß von 5 ml/min eingehalten wurde. Fraktionen mit maximaler Proteinkonzentration wurden gesammelt. Die Säule wurde mit Puffer A nachgespült. Es konnten 360ml eines klaren Kaninchenserums gewonnen werden, in dem kein Cholesterin mehr nachgewiesen werden konnte (<2,5mg/dl).

### 6. Elution der Lipoproteine

Eine mit Humanlipoproteinen beladene Säule nach Beispiel 4 wurde mit Puffer A gespült, bis im Eluat eine Basislinienabsorption erreicht wurde. Nun wurden die Lipoproteine von der Säule in einem Puffer, bestehend aus Puffer A mit 1% Triton^{R} X-100, eluiert. Fraktionen mit maximaler Absorption wurden gesammelt. Es konnte ein Lipoproteinkonzentrat gewonnen werden, das ca. 80% der aufgetragenen Lipoproteine in etwa 2facher Konzentration gegenüber dem aufgetragenen Plasma enthielt.

## Patentansprüche

1. Adsorbentien zur Entfernung von Lipoproteinen aus menschlichen oder tierischen Körperflüssigkeiten, bestehend aus einem zur Chromatographie geeigneten Polysaccharid-Träger, vorzugsweise auf der Basis von Agarose, der in einer chemischen Reaktion mit einem Glycidylether eines nichionischen Polyoxyethylen-Detergenz des Typs HO-(CH₂-CH₂-O)ₙ-Y-R umgesetzt wurde.

2. Adsorbens nach Anspruch 1 mit der Formel I 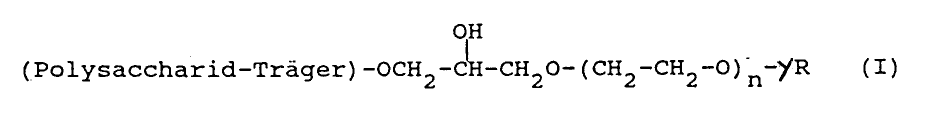 wobei
n eine ganze Zahl von 2 bis 30 und
R ein Alkylrest mit 4-20 Kohlenstoffatomen oder ein Phenyl-Rest oder ein Phenyl-Alkyl-Rest ist, wobei der Alkylrest 1 - 16 Kohlenstoffatome hat.

3. Adsorbens nach Anspruch 2, dadurch gekennzeichnet, daß n eine ganze Zahl von 6 bis 20 ist.

4. Adsorbens nach Anspruch 2, dadurch gekennzeichnet, daß R ein Alkylrest mit 10 - 16 Kohlenstoffatomen ist.

5. Adsorbens nach Anspruch 2, dadurch gekennzeichnet, daß das Träger-Polysaccharid ein makroporöses Agarose-Gel mit einem Agarosegehalt von 1 - 6 % ist.

6. Verfahren zur Herstellung eines Adsorbens der Formel I in Anspruch 2, dadurch gekennzeichnet, daß ein Detergenz der Formel III,
HO-(CH₂CH₂O)ₙ-Y-R (III)
wobei
n eine ganze Zahl von 2 bis 30 und
R ein Alkylrest mit 4 - 20 Kohlenstoffatomen, ein Phenyl-Rest oder ein Phenyl-Alkyl-Rest ist, wobei der Alkylrest 1 - 16 Kohlenstoffatome hat, mit Epichlorhydrin zu einem Glycidylether der Formel II 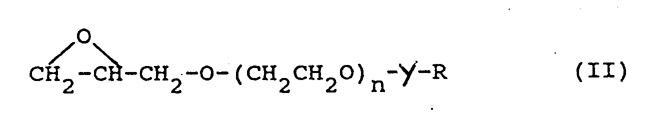 wird und dieser Glycidylether in Gegenwart einer Lewis-Säure mit einem Polysaccharid-Träger zu einer Verbindung der Formel I umgesetzt wird.

7. Verwendung eines Adsorbens nach Anspruch 1 zur Adsorption von Lipoproteinen.

## Claims

1. An adsorbent for the removal of lipoproteins from human or animal body fluids, composed of a polysaccharide support which is suitable for chromatography and is preferably based on agarose which has undergone a chemical reaction with a glycidyl ether of a nonionic polyoxyethylene detergent of the type HO-(CH₂-CH₂-O)ₙ--R.

2. An adsorbent as claimed in claim 1 having the formula I 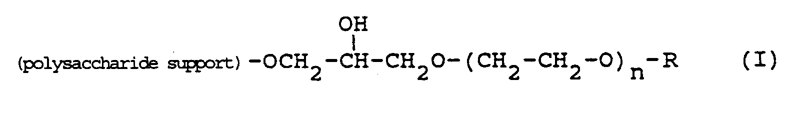 where
n is an integer from 2 to 30 and
R is an alkyl radical having 4-20 carbon atoms or a phenyl radical or a phenylalkyl radical, where the alkyl radical has 1-16 carbon atoms.

3. An adsorbent as claimed in claim 2, wherein n is an integer from 6 to 20.

4. An adsorbent as claimed in claim 2, wherein R is an alkyl radical having 10-16 carbon atoms.

5. An adsorbent as claimed in claim 2, wherein the support polysaccharide is a macroporous agarose gel with an agarose content of 1-6%.

6. A process for the preparation of an adsorbent of the formula I in claim 2, which comprises reacting a detergent of the formula III
HO-(CH₂CH₂O)ₙ--R (III)
where
n is an integer from 2 to 30 and
R is an alkyl radical having 4-20 carbon atoms, a phenyl radical or a phenylalkyl radical, where the alkyl radical has 1-16 carbon atoms, with epichlorohydrin to give a glycidyl ether of the formula II 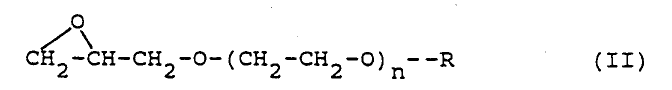 and reacting this glycidyl ether in the presence of a Lewis acid with a polysaccharide support to give a compound of the formula I.

7. The use of an adsorbent as claimed in claim 1 for the adsorption of lipoproteins.

## Revendications

1. Adsorbants pour l'élimination des lipoprotéines de liquides corporels humains ou animaux, constitué d'un support polysaccharide approprié à la chromatographie, de préférence à base d'agarose, que l'on a fait réagir dans une réaction chimique avec un éther glycidylique d'un détergent polyoxyéthylène non ionique du type HO-(CH₂CH₂O)ₙ-R.

2. Adsorbant selon la revendication 1 ayant la formule I 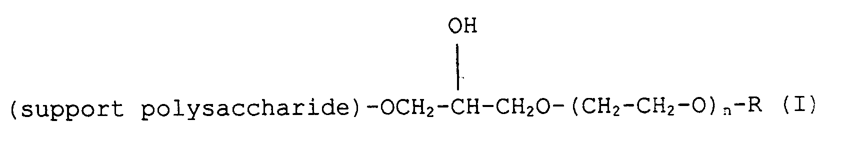 dans laquelle
n est un nombre entier compris entre 2 et 30 et
R est un groupe alkyle avec entre 4 et 20 atomes de carbone ou un groupe phényle ou un groupe phénylalkyle, le groupe alkyle ayant entre 1 et 16 atomes de carbone.

3. Adsorbant selon la revendication 2, caractérisé en ce que n est un nombre entier compris entre 6 et 20.

4. Adsorbant selon la revendication 2, caractérisé en ce que R est un groupe alkyle avec entre 10 et 16 atomes de carbone.

5. Adsorbant selon la revendication 2, caractérisé en ce que le support de polysaccharide est un gel d'agarose macroporeux avec une teneur en agarose comprise entre 1 et 6%.

6. Procédé de préparation d'un adsorbant de la formule I de la revendication 2, caractérisé en ce que l'on fait réagir un détergent de formule III
HO-(CH₂CH₂O)ₙ-R (III)
dans laquelle
n est un nombre entier compris entre 2 et 30 et
R est un groupe alkyle avec entre 4 et 20 atomes de carbone, un groupe phényle ou un groupe phénylalkyle, le groupe alkyle ayant entre 1 et 16 atomes de carbone, avec l'épichlorhydrine, pour obtenir un éther glycidylique de formule II 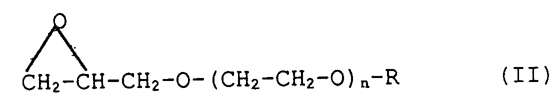 et en ce que l'on fait réagir cet éther glycidylique en présence d'un acide de Lewis avec un support de polysaccharide pour obtenir un composé de formule I.

7. Utilisation d'un adsorbant selon la revendication 1 pour l'adsorption de lipoprotéines.
